# EUROPÄISCHE PATENTANMELDUNG

(11) **EP 3 437 693 A1**
(43) Veröffentlichungstag der Anmeldung: **06.02.2019**
(21) Anmeldenummer: 17184421.0
(22) Anmeldetag: 02.08.2017
(51) Int. Cl.: A61N 1/375, A61N 1/362, A61N 1/38

(54) **ANSCHLUSSSTIFT UND DURCHFÜHRUNG SOWIE VERFAHREN ZUR HERSTELLUNG EINES ANSCHLUSSSTIFTES**

(71) Anmelder: BIOTRONIK SE & Co. KG, 12359 Berlin (DE)
(72) Erfinder: Müller, Teresa, 91054 Erlangen (DE); Sontheimer, Thomas, 90574 Roßtal (DE); Eck, Stefan, 91315 Höchstadt (DE)
(74) Vertreter: Keck, Hans-Georg

(57) **Zusammenfassung**

Anschlussstift einer Durchführung eines implantierbaren medizinelektronischen Gerätes, mit einem im Wesentlichen zylindrischen Stiftkörper und mindestens einem abgeflachten Endabschnitt, an dem mindestens eine ebene Anbindungsfläche, insbesondere zur stoffschlüssigen Anbindung eines bandförmigen Leiters, gebildet ist.

## Beschreibung

Die Erfindung betrifft einen Anschlussstift einer Durchführung eines implantierbaren medizinelektronischen Gerätes, eine einen solchen Anschlussstift umfassende Durchführung sowie ein Verfahren zur Herstellung eines solchen Anschlussstiftes.

Die meisten praktisch bedeutsamen implantierbaren medizinischen Geräte (IMDs) sind dazu vorgesehen, über geeignet platzierte Elektroden elektrische Impulse an reizbares Körpergewebe abzugeben. Ferner können viele Geräte elektrische Impulse und Reize im Körper des Patienten gezielt messen und über einen längeren Zeitraum aufzeichnen oder auswerten, um eine individuell angepasste Therapie zu wählen und den Erfolg der Behandlung in vivo zu überprüfen.

Um diese Funktionen auszuführen, sind im Gehäuse des Gerätes elektronische/elektrische Funktionseinheiten zur Erzeugung und Messung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Diese Aufgabe wird von sogenannten Durchführungen übernommen, die Gegenstand zahlreicher und höchst unterschiedlicher Entwicklungen gewesen sind. Die Aufgabe einer Durchführung besteht darin, die elektrischen Signale durch das hermetisch geschlossene Gehäuse zu führen und so einen elektrischen Kontakt der Elektronik im hermetisch dichten Gehäuse mit den Elektroden im Körper des Patienten zu ermöglichen. In vielen solchen Durchführungen werden hierfür Anschlussstifte eingesetzt, die im Geräteinneren mit der dort befindlichen Leiterplatte oder einem ähnlichen Leitungsträger kontaktiert werden und die Signale durch das Gehäuse führen.

Die US 7,747,321 B2 offenbart, wie in Fig. 1 gezeigt, einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet. Sie umfassen einen Draht-Kern, etwa aus Tantal, Niob, Titan, Molybdän oder Kupfer und eine oxidationsbeständige Ummantelung aus einem biokompatiblem Material, etwa Gold, Platin, Titan oder ähnlichem.

In bekannten Durchführungskonstruktionen werden als Anschlusselemente Stift-Bändchen-Konstruktionen benutzt, bei denen ein langgestrecktes Anschlusselement (Stift) einen Isolationskörper der Durchführung durchstößt und zum Anschweißen an ein Kopfteil des IMD genutzt wird, während ein auf das gehäuse-seitige Ende des Stiftes aufgeschweißtes Bändchen den Kontakt zur Geräteelektronik (Leiterplatte) herstellt. Der Stift kann aus Materialien wie Nb oder PtIr und das Bändchen aus Cu oder Ni bestehen, der Stift ist im Isolationskörper hart-verlötet, und das Bändchen ist typischerweise ebenfalls hart auf das Ende des Stiftes gelötet oder über einen Laserschweißpunkt mit ihm verbunden.

In vielen medizinelektronischen Geräten werden Anschlussstifte eingesetzt, die einen sogenannten Nail Head haben, der durch Kaltumformung eines gezogenen Drahtmaterials ausgebildet wird. Derartige Anschlussstifte sind in Folge der Kaltumformung typischerweise in ihrem Gefüge in nachteiliger Weise verändert und zeigen häufig Mikrorisse in der Oberfläche. Hieraus ergeben sich Probleme bei der Beschichtung, die für die Anbindung von Leitern durch einen Lötprozess notwendig ist. Zudem kann bei derartigen Anschlussstiften die Oberflächenqualität durch Ziehriefen beeinträchtigt sein, die insbesondere zu einem unerwünschten Fließen von Verbindungslot längs des Anschlussstiftes führen können.

Der Erfindung liegt die Aufgabe zu Grunde, einen Anschlussstift bereitzustellen, der in Bezug auf die Anbindung eines insbesondere bändchenförmigen Leiters mittels eines stoffschlüssigen Verbindungsprozesses verbesserte Eigenschaften aufweist. Weiterhin soll ein geeignetes Verfahren zur Herstellung eines solchen Anschlussstiftes angegeben werden.

Diese Aufgabe wird in ihrem Vorrichtungsaspekt durch einen Anschlussstift mit den Merkmalen des Anspruchs 1 und in ihrem Verfahrensaspekt durch ein Verfahren mit den Merkmalen des Anspruchs 8 gelöst. Zweckmäßige Fortbildung des Erfindungsgedankens sind Gegenstand der abhängigen Ansprüche.

Die Erfindung schließt den Gedanken ein, gewisse grundsätzliche Nachteile der bekannten Anschlussstifte hinsichtlich ihrer Anbindung durch eine Veränderung der Oberflächengeometrie mindestens in einem Endabschnitt des Stiftes zu überwinden. Sie schließt weiter den Gedanken ein, im relevanten Endabschnitt - und nur dort - eine zu bändchenförmigen Verbindungsleitern optimal passfähige Oberflächengeometrie zu erzeugen. Schließlich mündet die Erfindung in den Gedanken, am Stiftkörper mindestens einen abgeflachten Endabschnitt zu erzeugen, an dem mindestens eine ebene Anbindungsfläche, insbesondere zur stoffschlüssigen Anbindung eines bandförmigen Leiters, gebildet ist.

In einer Ausführungsform weisen beide Endabschnitte des Stiftkörpers einen abgeflachten Endabschnitt auf, an dem mindestens eine ebene Anbindungsfläche gebildet ist. In einer hierzu alternativen Ausführung ist an einem Ende des Stiftkörpers ein Nail Head ausgebildet, während das andere Ende erfindungsgemäß konfiguriert ist.

In weiteren Ausführungen der Erfindung ist vorgesehen, dass der oder jeder abgeflachte Endabschnitt zwei ebene Anbindungsflächen aufweist, die insbesondere einander gegenüber liegende Oberflächen des Endabschnitts bilden. Diese Ausführung bietet eine höhere Entwurfs-Freiheit hinsichtlich des geometrischen Verlaufes von Leitern, die an den Anschlussstift anzubinden sind.

In weiteren Ausführungen ist der oder mindestens ein abgeflachter Endabschnitt in einem Winkel im Bereich zwischen 30° und 90°, insbesondere in einem Winkel von 90°, zur Längsachse des zylindrischen Stiftkörpers umgebogen. Diese Umbiegung bietet zusätzliche Freiheitsgrade hinsichtlich des Verlaufes des stift-nahen Endabschnittes eines anzubindenden Leiter-Bändchens und ermöglicht gegebenenfalls eine kompaktere Ausführung der Durchführung.

In weiteren Ausführungen weist die oder jede Anbindungsfläche eine, insbesondere im Wesentlichen quer zur Längsachse ausgerichtete oder irreguläre, Oberflächenstruktur auf, die das Verlaufen eines Verbindungsmaterials zur stoffschlüssigen Anbindung eines bandförmigen Leiters in Längsachsen-Richtung behindert. Hierdurch lässt sich in vorteilhafter Weise ein erheblicher herstellungsbedingter Nachteil von aus gezogenem Draht hergestellten Anschlussstiften kompensieren oder sogar über-kompensieren.

Unter Verfahrensaspekten schließt die Erfindung den Gedanken ein, bei der Herstellung des Anschlussstiftes von einem spannungsarmen, insbesondere geglühten Drahtmaterial auszugehen, und hierdurch die Möglichkeit des Einsatzes eines Laserbearbeitungsverfahrens zur Erzeugung der erfindungsgemäßen Endabschnittkonfiguration zu schaffen. Weiter gehört zum Verfahrensaspekt der Erfindung der Gedanke, diese Endabschnitt-Konfiguration durch eine Ultrakurzpuls- (UKP-) Laserbehandlung zu erzeugen.

In einer Ausführung des Verfahrens wird zusammenhängend mit der Ausbildung des abgeflachten Endabschnittes eine für die Anbindung optimierte Oberflächenstruktur auf der oder jeder Anbindungsfläche, bevorzugt durch die UKP-Laserbehandlung ausgebildet.

Dies kann durch eine gezielt auf den Fügepartner angepasste Oberflächenstruktur oder durch eine gezielt gerichtete Oberflächenrauigkeit erfolgen.

Dies trägt sowohl zu einer effizienten Herstellung des Anschlussstiftes mit hoher Produktionsausbeute, als auch zu einer dauerhaften und langzeitstabilen Kontaktierung bei.

In weiteren Ausführungen wird der Anschlussstift nach Ausbildung des oder jedes abgeflachten Endabschnittes in eine Durchführung eines implantierbaren medizinelektronischen Gerätes verbaut und der geräteinnere Endabschnitt im verbauten Zustand durch Kaltumformung umgebogen. Diese Schrittabfolge vereinfacht die Realisierung von Durchführungen mit umgebogenen Anschlussstiften und schafft unter gewissen Umständen überhaupt erst die Voraussetzung für die Herstellung solcher Durchführungen. In einer Ausgestaltung hiervon ist vorgesehen, dass der geräteäußere Endabschnitt des Anschlussstiftes vor dem Verbau in die Durchführung durch Kaltumformung umgebogen wird.

Mit der Erfindung lassen sich, jedenfalls in gewissen Ausführungsformen, mindestens einige der folgenden Vorteile gegenüber bekannten Anschlussstiften realisieren:
- Es wird eine erhöhte Flexibilität in Bauteilgeometrie, Materialauswahl und Produktanwendungen geboten.
- Es wird eine erhöhte Entwurfs-Freiheit hinsichtlich der Anordnung und geometrischen Konfiguration der Leiter-Verbindungen zu den Gerätekomponenten geboten.
- Das vorgeschlagene Verfahren ist gut automatisierbar und parametrisierbar und kommt weitgehend ohne Umrüstschritte aus.
- Es ist möglich, bei der stoffschlüssigen Anbindung der Leiterbändchen Prozessschritte und Kosten zu sparen.
- Qualitäts- und Zuverlässigkeitsprobleme in Folge von Oberflächen- oder Gefügemängeln im Bereich der Anbindungsflächen werden weitestgehend vermieden.
- In Abhängigkeit von Anwendungsszenarien kann gegebenenfalls auf eine mehrteilige Pads-Pin-Konfiguration verzichtet werden und es können entsprechende Einsparungen an Herstellungszeit und -kosten realisiert werden.
- Die Prozesssicherheit im Schritt der Anbindung von bandförmigen Leitern an den Kontaktstift wird signifikant erhöht.

Vorteile und Zweckmäßigkeiten der Erfindung ergeben sich im Übrigen aus der nachfolgenden Beschreibung eines Ausführungsbeispiels anhand der Figuren. Von diesen zeigen:

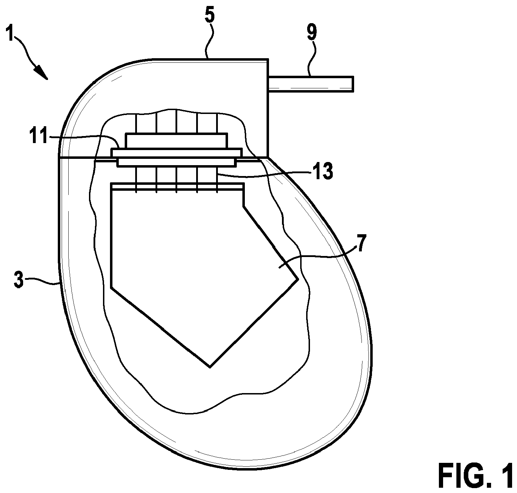

- Fig. 1: eine schematische Darstellung eines herkömmlichen Herzschrittmachers,
- Fig. 2: eine erste Ausführungsform des erfindungsgemäßen Anschlussstiftes,
- Fig. 3: eine zweite Ausführungsform des erfindungsgemäßen Anschlussstiftes,
- Fig. 4: eine dritte Ausführungsform des erfindungsgemäßen Anschlussstiftes, und
- Fig. 5: eine vierte Ausführungsform des erfindungsgemäßen Anschlussstiftes.

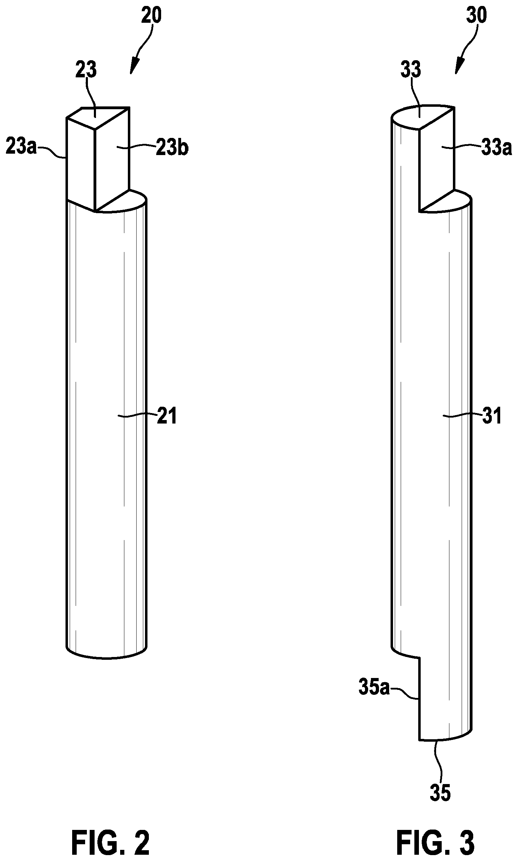

Fig. 2 zeigt in einer perspektivischen Darstellung einen Anschlussstift 20 mit einem zylindrischen Stiftkörper 21, der den überwiegenden Teil der Längserstreckung des Anschlussstiftes bildet, und einem in Art eines Prismas verformten Endabschnitt 23, der zwei gegenüberliegende ebene Anbindungsflächen 23a, 23b, zum Anschweißen eines (nicht gezeigten) Leiterbändchens bietet. Die geometrische Konfiguration des Endabschnitts 23 kann durch eine UKP-Laserbearbeitung oder gegebenenfalls auch durch einen mechanischen Umformschritt ausgeprägt werden.

Fig. 3 zeigt als weiteres Ausführungsbeispiel einen Anschlussstift 30, der wiederum einen sich über den größten Teil der Längserstreckung des Anschlussstiftes ausdehnenden zylindrischen Stiftkörper 31 hat, an dessen gegenüberliegenden Enden aber zwei deformierte Endabschnitte 33, 35 durch abtragende Bearbeitung (beispielsweise wieder mittels eines UKP-Laser-Verfahrens) ausgebildet sind. In den Endabschnitten 33, 35 ist die zylindrische Grundform über den größten Teil des Querschnitts erhalten, durch die abtragende Bearbeitung ist jedoch ein Abschnitt des Zylinders entfernt und jeweils eine plane Anbindungsfläche 33a bzw. 35a zum Anschweißen eines Leiterbändchens gebildet.

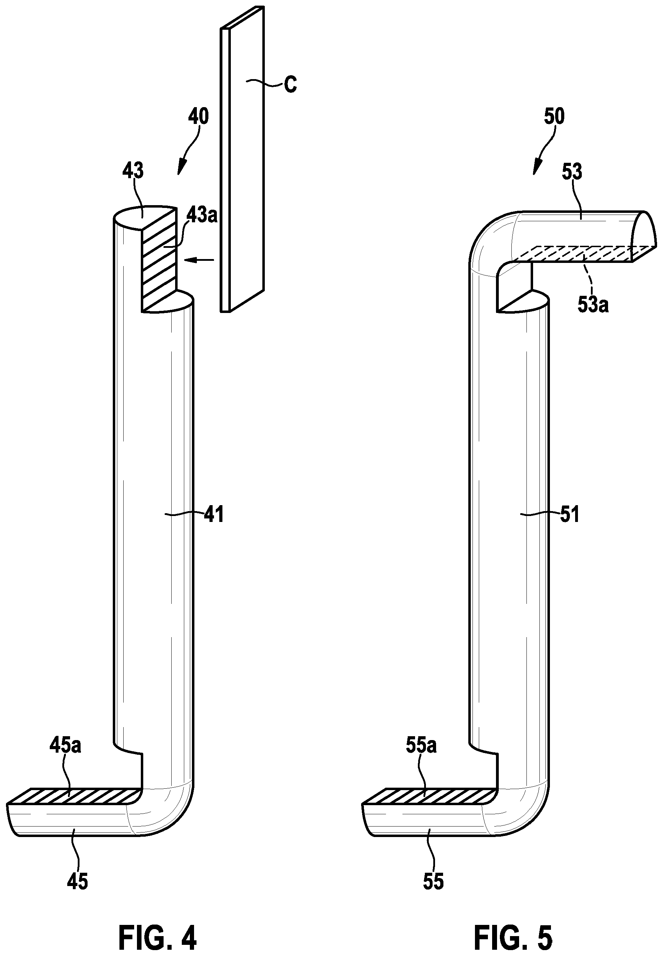

Fig. 4 zeigt als weitere Ausführungsform der Erfindung einen Anschlussstift 40, der wiederum einen im Wesentlichen zylindrischen Stiftkörper 41 umfasst, zusammen mit einem Leiterbändchen C. Auch bei diesem Anschlussstift 40 sind beide Endabschnitte 43, 45 derart mit einem abtragenden Verfahren bearbeitet, dass eine plane Anbindungsfläche 43a bzw. 45a geschaffen ist. Die Anbindungsflächen 43a, 45a sind überdies durch quer zur Längserstreckung des Anschlussstiftes verlaufende Mikrorillen bzw. -riefen derart strukturiert, dass ein Verlaufen von Verbindungslot in Längsrichtung des Anschlussstiftes behindert wird. Überdies ist bei dieser Ausführung der Endabschnitt 45 rechtwinklig zur Längsachse des Anschlussstiftes umgebogen. Das Leiterbändchen C wird an die Anbindungsfläche 43a angeschweißt oder -gelötet, und analog kann an die Anbindungsfläche 45a ein weiteres (nicht gezeigtes) Leiterbändchen mittels einer stoffschlüssigen Verbindung angebracht werden.

Fig. 5 zeigt als weitere Ausführungsform einen Anschlussstift 50 in einer Konfiguration, die er nach Verbau in einer (nicht gezeigten) Durchführung einnimmt, bei der nämlich beide an den Stiftkörper 51 angrenzende Endabschnitte 53, 55 rechtwinklig zur Stift-Längsachse umgebogen sind. Die Anbindungsflächen 53a, 55a sind hier mikro-rau ausgeführt, was ähnlich wie bei der Ausführung nach Fig. 4 ein Verfließen von Lotmaterial in Längsrichtung des Anschlussstiftes behindert.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Anschlussstift (20, 30, 40, 50) einer Durchführung (11) eines implantierbaren medizinelektronischen Gerätes (1), mit einem im Wesentlichen zylindrischen Stiftkörper (21; 31; 41; 51) und mindestens einem abgeflachten Endabschnitt (23; 33, 35; 43, 45; 53, 55), an dem mindestens eine ebene Anbindungsfläche (23a, 23b; 33a, 35a; 43a, 45a; 53a, 55a), insbesondere zur stoffschlüssigen Anbindung eines bandförmigen Leiters (C), gebildet ist.

2. Anschlussstift nach Anspruch 1, wobei beide Enden des Stiftkörpers (31; 41; 51) einen abgeflachten Endabschnitt (33, 35; 43, 45; 53, 55) aufweisen, an dem mindestens eine ebene Anbindungsfläche (33a, 35a; 43a, 45a; 53a, 55a) gebildet ist.

3. Anschlussstift nach Anspruch 1, wobei an einem Ende des Stiftkörpers ein Nail Head ausgebildet ist.

4. Anschlussstift nach einem der vorangehenden Ansprüche, wobei der oder jeder abgeflachte Endabschnitt (23) zwei ebene Anbindungsflächen (23a, 23b) aufweist, die insbesondere einander gegenüber liegende Oberflächen des Endabschnitts bilden.

5. Anschlussstift nach einem der vorangehenden Ansprüche, wobei der oder ein abgeflachter Endabschnitt (45; 55) in einem Winkel im Bereich zwischen 30° und 90°, insbesondere in einem Winkel von 90°, zur Längsachse des zylindrischen Stiftkörpers (41; 51) umgebogen ist.

6. Anschlussstift nach einem der vorangehenden Ansprüche, wobei die oder jede Anbindungsfläche (23a, 23b; 33a, 35a; 43a, 45a; 53a, 55a) eine, insbesondere im Wesentlichen quer zur Längsachse ausgerichtete oder irreguläre, Oberflächenstruktur aufweist, die das Verlaufen eines Verbindungsmaterials zur stoffschlüssigen Anbindung eines bandförmigen Leiters (C) in Längsachsen-Richtung behindert.

7. Durchführung (11) eines implantierbaren medizinelektronischen Gerätes, insbesondere eines Herzschrittmachers oder Kardioverters, die mindestens einen Anschlussstift nach einem der Ansprüche 1 bis 6 aufweist.

8. Verfahren zur Herstellung eines Anschlussstiftes (20, 30, 40, 50) nach einem der Ansprüche 1 bis 6, wobei der Stiftkörper (21; 31; 41; 51) aus einem geglühten Drahtmaterial erzeugt und zur Ausbildung des oder jedes abgeflachten Endabschnittes (23; 33, 35; 43, 45; 53, 55) einer Ultrakurzpuls- (UKP-) Laserbehandlung unterzogen wird.

9. Verfahren nach Anspruch 8, wobei zusammenhängend mit der Ausbildung des abgeflachten Endabschnittes (23; 33, 35; 43, 45; 53, 55) eine im Wesentlichen quer zur Längsachse ausgerichtete Oberflächenstruktur auf der oder jeder Anbindungsfläche (23a, 23b; 33a, 35a; 43a, 45a; 53a, 55a) durch die UKP-Laserbehandlung ausgebildet wird.

10. Verfahren nach Anspruch 8 oder 9, wobei der Anschlussstift (20, 30, 40, 50) nach Ausbildung des oder jedes abgeflachten Endabschnittes (23; 33, 35; 43, 45; 53, 55) in eine Durchführung eines implantierbaren medizinelektronischen Gerätes verbaut und der geräteinnere Endabschnitt (45; 55) im verbauten Zustand durch Kaltumformung umgebogen wird.

11. Verfahren nach Anspruch 10, wobei der geräteäußere Endabschnitt (53) des Anschlussstiftes vor dem Verbau in die Durchführung durch Kaltumformung umgebogen wird.
